# EUROPEAN PATENT APPLICATION

(11) **EP 4 834 400 A1**
(43) Date of publication of application: **04.11.2026**
(21) Application number: 24910622.0
(22) Date of filing: 05.12.2024
(51) Int. Cl.: A61M 16/06

(54) **MASK SYSTEM, VENTILATION DEVICE, AND METHOD FOR MANUFACTURING MASK**

(30) Priority: 29.12.2023 CN 202311865785
(71) Applicant: BMC Medical Co., Ltd., Beijing 100089 (CN); BMC (Tianjin) Medical Co., Ltd., Tianjin 301700 (CN)
(72) Inventor: LIU, Lina, Beijing 100089 (CN); ZHOU, Mingzhao, Beijing 100089 (CN); WANG, Yajie, Beijing 100089 (CN); LIU, Chunxiu, Beijing 100089 (CN); ZHUANG, Zhi, Beijing 100089 (CN)
(74) Representative: Grünecker Patent- und Rechtsanwälte PartG mbB
(86) International application number: PCT/CN2024/137229
(87) International publication number: WO 2025/139690

(57) **Abstract**

The present application relates to the technical field of respiratory apparatuses, and in particular to a mask system, a ventilation device, and a mask manufacturing method. The mask system includes a structural member and a fabric. The fabric is formed from yarns on at least a portion of an outer surface of the structural member. The fabric and the structural member as a whole are structurally stable and provide corresponding functions, and can also provide a user with good comfort and breathability when the structural member is in contact with a human body, thereby improving user satisfaction. The fabric has no protruding interfaces, and the fabric and the structural member as a whole have no protruding areas, thereby avoiding discomfort to the face or the head of the user. Moreover, when the structural member has a complex shape, the yarns can still be formed into a fabric on the outer surface of the structural member, so that the styling and use requirements of the mask system can be met.

## Description

### Cross Reference to Related Application

The present application claims the priority to Chinese Patent Application No. 202311865785.4, filed with the China National Intellectual Property Administration on December 29, 2023 and entitled "MASK SYSTEM, VENTILATION DEVICE, AND METHOD FOR MANUFACTURING MASK", which is incorporated herein by reference in its entirety.

### Technical Field

The present invention relates to the technical field of respiratory apparatuses, and in particular to a mask system, a ventilation device, and a method for manufacturing mask.

### Background

A mask system is worn in a relatively comfortable area of the face by means of a headstrap assembly, thereby ensuring the comfort of wearing the mask system and further better realizing the regulation, improvement, and therapeutic functions of the mask system. The mask system generally includes a piece of cloth, a silicone member or a plastic member. The cloth has the advantages of good comfort and breathability. Since untreated cloth is generally unable to withstand pressurized gas, the cloth serves as part of a positioning and stabilizing assembly, namely the headstrap assembly. Alternatively, the cloth serves as an outer covering for the silicone member or the plastic member to provide a user with good comfort and breathability.

The cloth wrapping around the silicone member or the plastic member can be connected in either a removable or a non-removable manner. The removable connection is achieved by means of adhesives, buttons, zippers, direct insertion, etc. Using the adhesives, the buttons, or the zippers requires the provision of corresponding components on the cloth, and the cloth thus will have protruding areas that are likely to cause discomfort to the face or the head. Direct insertion of the silicone member or the plastic member into the cloth imposes high requirements on the complexity of the components and is therefore suitable only for components with simple structural shapes. The non-removable connection relies on the specific assembly sequence of the components, with the cloth directly serving as part of the assembly. This is the equivalent of splitting components that can be integrally formed into multiple components to be assembled with the cloth, resulting in complex manufacturing. In the prior art, the cloth may also be integrally moulded with the silicone member or the plastic member. This integrally moulded member may be used to manufacture a strut arm of a frame assembly or the headstrap assembly. Such a product has the advantages of good comfort and high breathability during use. However, the integral moulding of the cloth with the silicone member or the plastic member requires very high process technology, incurs high costs, and is not easy to implement.

### Summary

Therefore, the present invention provides a mask system, a ventilation device, and a mask manufacturing method, in order to solve or partially solve the problems in the prior art. Specifically, when cloth wraps around a silicone member or a plastic member, there are protruding areas which are likely to cause discomfort to the face or the head, and thus there are stringent requirements on the shape of the silicone member or the plastic member. Furthermore, when the cloth and the silicone member or the plastic member are manufactured by integral moulding, the manufacturing process requires very high process technology, incurs high costs, and is not easy to implement.

In a first aspect, an embodiment of the present invention provides a mask system, including a fabric and at least one structural member, where the structural member is at least a partial structure of the mask system, and at least a portion of an outer surface of the structural member is disposed opposite to a human skin in an in-use state; and the fabric is formed from yarns on at least a portion of the outer surface of the structural member, and the structural member is in contact with the human skin via the fabric.

Optionally, the structural member comprises a first support portion configured to be in contact with a human face; and the fabric wraps around an outer surface of the first support portion.

Optionally, the structural member is provided with a connecting portion configured to be connected to an external member; and the fabric is provided with a clearance opening for providing clearance for the connecting portion.

Optionally, the connecting portion is at least one of a ventilation port, an ear strap portion, a connection port and an electrical interface.

Optionally, the structural member is at least one of a silicone member and a plastic member.

Optionally, the structural member is at least one of a bracket, a frame and a ventilation headstrap tube.

Optionally, the fabric is a knitted member.

Optionally, the fabric is a woven member.

In a second aspect, an embodiment of the present invention provides a ventilation device, including a ventilator and a mask system, where the ventilator is connected to the mask system, and the mask system is a mask system described above.

In a third aspect, an embodiment of the present invention provides a mask manufacturing method for use in manufacturing a mask system described above, the manufacturing method including: providing a structural member; and forming yarns into a fabric on at least a portion of an outer surface of the structural member.

Optionally, providing a structural member includes: fixing the structural member; and providing a support mold in the structural member, with the structural member being supported by the support mold to assume an in-use state.

Optionally, fixing the structural member includes: fixing the structural member to a worktable of a fabric forming device, the fabric forming device being configured to form the yarns into a fabric.

Optionally, forming yarns into a fabric on at least a portion of an outer surface of the structural member includes: providing a 3D model of the structural member in an in-use state; and forming yarns into a fabric on at least a portion of an outer surface of the structural member based on the 3D model.

Optionally, the yarns are knitted on at least a portion of the outer surface of the structural member.

Optionally, the yarns are woven on at least a portion of the outer surface of the structural member.

In the embodiments of the present invention, the fabric is formed from yarns on at least a portion of the outer surface of the structural member, and has the advantages of good comfort and breathability. In this way, the fabric and the structural member as a whole are structurally stable and provide corresponding functions, and can also provide a user with good comfort and breathability when the structural member is in contact with a human body, thereby improving user satisfaction. The fabric has no protruding interfaces, and the fabric and the structural member as a whole have no protruding areas, thereby avoiding discomfort to the face or the head of the user. Moreover, when the structural member has a complex shape, the yarns can still be formed into a fabric on the outer surface of the structural member, so that the shape of the mask system and the shape of the structural member can be made very complex to meet the styling and use requirements of the mask system. The production process of forming the yarns into a fabric on at least a portion of the outer surface of the structural member can be carried out at ambient temperature. Compared with the method of integrally moulding the cloth with the silicone member or the plastic member, this process has the advantages of low production costs, reduced process difficulty, easier manufacturability, and suitability for mass production.

Furthermore, the fabric only wraps around the outer surface of the structural member and is not connected to the structural member by, for example, welding or moulding, thereby making the structure of the mask system simpler. By forming the fabric on only a portion of the outer surface of the structural member, the production cost and production time of the mask system can be reduced.

In the mask system of the embodiments of the present invention, the thickness and stiffness of the fabric can be set according to usage requirements, and the thickness and stiffness of the fabric in different regions on the same structural member may also be designed to be different, so that different areas have different breathability while meeting the rigidity requirements of the structural member.

The above-mentioned description is merely a summary of the technical solutions of the present invention. In order to more clearly understand the technical means of the present invention so that the implementation can be carried out according to the content of the description, and in order to make the above-mentioned and other objects, features and advantages of the present invention more comprehensible, the particular embodiments of the present invention will be illustrated below.

### Brief Description of the Drawings

In order to illustrate the technical solutions of the embodiments of the present invention more clearly, the accompanying drawings required for describing the embodiments of the present invention will be briefly introduced below. It is clear that the accompanying drawings in the description below show only some embodiments of the present invention, and a person of ordinary skill in the art can obtain other accompanying drawings according to these accompanying drawings without creative efforts.
Fig. 1 shows a structural schematic view of a ventilation headstrap tube in an embodiment of the present invention;
Fig. 2 shows a structural schematic view of a ventilation headstrap tube in another embodiment of the present invention;
Fig. 3 shows a structural schematic view of a ventilation headstrap tube and a fabric connected to each other in an embodiment of the present invention;
Fig. 4 is a structural schematic front view of the ventilation headstrap tube and the fabric connected to each other in Fig. 3;
Fig. 5 is a structural schematic left view of the ventilation headstrap tube and the fabric connected to each other in Fig. 3;
Fig. 6 is a structural schematic right view of the ventilation headstrap tube and the fabric connected to each other in Fig. 3;
Fig. 7 shows a structural schematic view of a ventilation headstrap tube and a fabric connected to each other in another embodiment of the present invention;
Fig. 8 shows a schematic flow chart of a mask manufacturing method in an embodiment of the present invention; and
Fig. 9 shows a schematic flow chart of a mask manufacturing method in another embodiment of the present invention.

### Detailed Description of Embodiments

The technical solutions in the embodiments of the present invention will be clearly and completely described below in conjunction with the accompanying drawings in the embodiments of the present invention. Apparently, the embodiments described are some rather than all of the embodiments of the present invention. On the basis of the embodiments of the present invention, all the other embodiments obtained by a person of ordinary skill in the art without creative efforts shall fall within the scope of protection of the present invention.

An embodiment of the present application provides a mask system, which is configured to be worn on a human face and positioned at a relatively comfortable area of the face by means of a headstrap kit, thereby ensuring the user's comfort. The mask system includes various types and structures, and the type and the structure of the mask system are not specifically limited in the embodiments of the present application. For example, the mask system includes a nasal mask for covering only the nose; an oral mask for covering only the mouth; an oronasal mask for covering both the mouth and the nose, where the oronasal mask is also referred to as a full-face mask; and a nasal pillow mask inserted into the nostrils.

In an embodiment of the present application, the mask system includes a fabric 20 and at least one structural member. The structural member is at least a partial structure of the mask system, and at least a portion of an outer surface of the structural member is disposed opposite to a human skin in an in-use state. The fabric 20 is formed from yarns on at least a portion of the outer surface of the structural member, and the structural member is in contact with the human skin via the fabric 20.

The yarn is a kind of textile, which is a product of a specified fineness processed from various textile fibres, and used for cloth-forming, rope-making, thread-making, knitting, embroidery, etc. Yarns are classified into staple fibre yarns, continuous filaments, etc. A plurality of yarns are formed into a fabric after establishing a stable relationship, such that the fabric has the advantages of good comfort and breathability. The fabric can provide a user with good comfort and breathability when in contact with the human body.

The structural member has certain functions, which are not specifically limited in the embodiments of the present application. For example, the structural member is used to deliver gas. For example, the structural member is used to provide support such that a cavity is formed between the mask and the face. For example, the structural member is used to connect to a gas delivery tube. For another example, the structural member is a component, a ventilation headstrap tube 10, or a strut arm of the mask system.

In the mask system of the embodiments of the present application, the structural member may provide appropriate functions such as gas delivery and support functions, and the fabric 20 and the structural member as a whole may implement the gas delivery and support functions. The fabric 20 is formed from yarns on at least a portion of the outer surface of the structural member. The fabric 20 has the advantages of good comfort and breathability. In this way, the fabric 20 and the structural member as a whole are structurally stable and provide corresponding functions, and can also provide a user with good comfort and breathability when the structural member is in contact with the human body, thereby improving user satisfaction.

In the mask system of the embodiments of the present application, the yarns are formed into a fabric on at least a portion of the outer surface of the structural member, so that the fabric has no protruding interfaces, and the fabric 20 and the structural member as a whole have no protruding areas, thereby avoiding discomfort to the face or the head of the user. Moreover, when the structural member has a complex shape, the yarns can still be formed into a fabric on the outer surface of the structural member, so that the shape of the mask system and the shape of the structural member can be made very complex to meet the styling and use requirements of the mask system.

In the mask system of the embodiments of the present application, the production process of forming the yarns into a fabric on at least a portion of the outer surface of the structural member can be carried out at ambient temperature. Compared with the method of integrally moulding the cloth with the silicone member or the plastic member, this process has the advantages of low production costs, reduced process difficulty, easier manufacturability, and suitability for mass production.

Further, when the yarns are formed into a fabric on at least a portion of the outer surface of the structural member, the fabric 20 only wraps around the outer surface of the structural member and is not connected to the structural member by, for example, welding or moulding. The structural member provides support for the shaping of the fabric 20, and the structural member and the fabric 20 jointly serve as at least a partial structure of the mask system.

In the mask system of the embodiments of the present application, the thickness and stiffness of the fabric 20 can be set according to usage requirements, and the thickness and stiffness of the fabric 20 in different regions on the same structural member may also be designed to be different, so that different areas have different breathability while meeting the rigidity requirements of the structural member.

Optionally, in the mask system of the embodiments of the present application, the structural member includes a first support portion 11. The first support portion 11 is configured to be in contact with a human face. The fabric 20 wraps around an outer surface of the first support portion 11. In the above structure of the mask system, the fabric 20 wraps around the outer surface of the first support portion 11, such that the first support portion 11 is in contact with the human face via the fabric 20. Since the fabric 20 has the advantages of good comfort and breathability, when the first support portion 11 is in contact with the human face via the fabric 20, good comfort and breathability can be provided for the user, thereby improving user satisfaction.

Optionally, in the mask system of the embodiments of the present application, the structural member further includes a second support portion 12. The second support portion 12 is not in contact with the human face. The second support portion may be in contact with the hair of the human body, or may be not in contact with the human body. Since no fabric 20 is provided on the second support portion 12, the production cost and production time of the mask system can be reduced.

Optionally, in the mask system of the embodiments of the present application, the structural member is provided with a connecting portion configured to be connected to an external member; and the fabric 20 is provided with a clearance opening for providing clearance for the connecting portion. In the embodiments of the present application, the provision of the clearance opening in the fabric 20 enables the structural member to be connected to the external member via the connecting portion, so that the fabric 20 can wrap around the structural member and the structural member provides better comfort and breathability, without affecting the use of the structural member.

Optionally, the type and structure of the connecting portion are not specifically limited in the embodiments of the present application. The connecting portion is at least one of a ventilation port 13, an ear strap portion 14, a connection port 15 and an electrical interface.

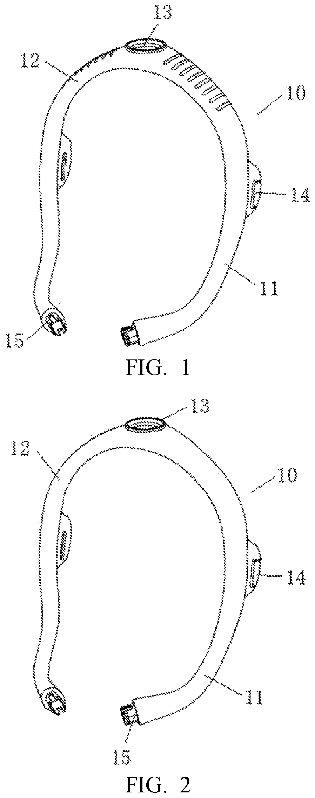

The type and structure of the connecting portion on the structural member vary depending on the type of the structural member. For example, referring to Figs. 1 and 2, when the structural member is a ventilation headstrap tube 10, a ventilation port 13 is provided at a top end of the ventilation headstrap tube 10. The ventilation port 13 is configured to be connected to an external gas pipe to implement a ventilation function of the ventilation headstrap tube 10. Ear strap portions 14 are provided on two lateral sides of the ventilation headstrap tube 10, and the ventilation headstrap tube 10 is held on the ears by means of the ear strap portions 14. A connection port 15 is provided at an end of the ventilation headstrap tube 10, and the ventilation headstrap tube 10 is connected to the external member via the connection port 15.

Optionally, in the mask system of the embodiments of the present application, the structural member is at least one of a silicone member and a plastic member.

Silicone, also known as silica gel, is a highly active adsorbent material and belongs to amorphous substances. Silicone has good biocompatibility, and is nonirritating, non-toxic, and non-allergenic to human tissue, with minimal rejection response from the body. Silicone possesses favourable physicochemical properties, and maintains its original elasticity and softness when in contact with body fluids and tissues without being degraded, making it a fairly stable inert substance. Silicone can withstand high temperatures and can be sterilized. Silicone is easy to process and mould, making it suitable for use in the mask system.

Plastic is mainly composed of carbon, oxygen, hydrogen, nitrogen, and other organic or inorganic elements. The finished product of plastic is solid, but during the manufacturing process, plastic is in a molten liquid state. Therefore, plastic can be heated to melt, pressurized to flow, and cooled to solidify, thereby forming various shapes. This large and diverse family of materials is called plastic. Plastic is also applicable for use in the mask system.

Optionally, in the mask system of the embodiments of the present application, the type and structure of the structural member are not specifically limited in the embodiments of the present application. For example, the structural member is at least one of a bracket, a frame and the ventilation headstrap tube 10.

Optionally, in the mask system of the embodiments of the present application, the fabric 20 is a knitted member.

Knitting is a process, specifically a process in which loops are formed from yarns of various materials and types using knitting needles, and are then connected to each other by interlooping to form a knitted fabric. The knitted fabric has a soft texture, good wrinkle resistance and breathability, and high extensibility and elasticity, making it comfortable to wear. Knitting is classified into hand knitting and machine knitting. In the mask system of the embodiments of the present application, the fabric 20 may be manufactured by either hand knitting or machine knitting of yarns.

Optionally, in the mask system of the embodiment of the present application, the fabric 20 is a woven member.

Weaving is a textile process, generally referring to machine weaving. Machine weaving refers to the process of forming a woven fabric by interlacing warp and weft yarns according to various fabric structures. The fabric 20 may be a woven member formed by weaving yarns.

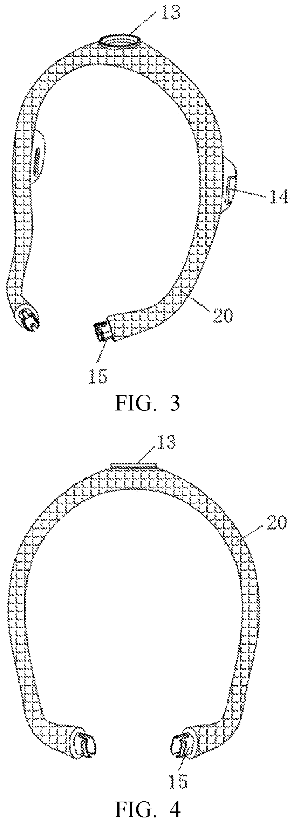

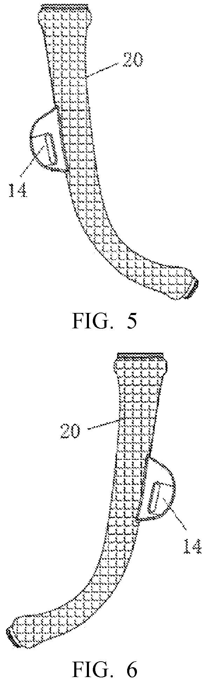

In a specific embodiment of the present application, referring to Figs. 1 and 2, the mask system includes a ventilation headstrap tube 10. The ventilation headstrap tube 10 is a silicone part integrally formed by injection moulding. This silicone part is a special-shaped part, which is hollow for ventilation and externally contacts the face and the head. The ventilation headstrap tube 10 is provided with a ventilation port 13, ear strap portions 14, and a connection port 15. The ventilation headstrap tube 10 takes air through the ventilation port 13, is hung on the ears by means of the ear strap portions 14, and is connected and fixed by means of the connection port 15. Referring to Figs. 3 to 6, the entire outer surface of the ventilation headstrap tube 10 is provided with the fabric 20, and the fabric 20 provides clearance for the ventilation port 13, the ear strap portions 14 and the connection port 15 during the forming of the yarns into the fabric. The ventilation headstrap tube 10 can achieve its corresponding functions by means of the ventilation port 13, the ear strap portions 14 and the connection port 15. Further referring to Figs. 1 and 2, an upper outer surface of the ventilation headstrap tube 10 shown in Fig. 1 is provided with a relief structure, while the upper outer surface of the ventilation headstrap tube 10 shown in Fig. 2 is smooth. Whether the outer surface of the ventilation headstrap tube 10 is provided with a relief structure does not affect the forming of the yarns into the fabric.

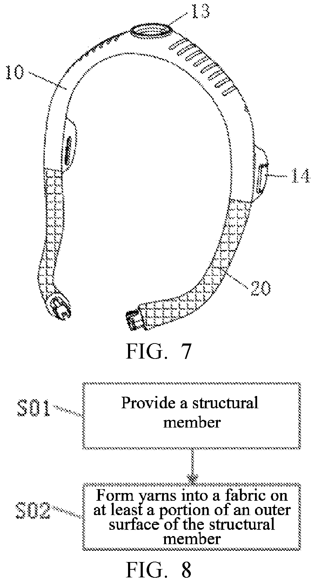

Referring to Fig. 7, a lower side of the ventilation headstrap tube 10 is configured for contact with the face, and the fabric 20 is arranged at a portion of the lower side of the ventilation headstrap tube 10 that is in contact with the face. The ventilation headstrap tube 10 is in contact with the face via the fabric 20, thereby providing the user with good comfort and breathability, and improving user satisfaction. Clearance is provided for the connection port 15 during the forming of the yarns into the fabric, and the ventilation headstrap tube 10 can achieve its corresponding function by means of the connection port 15.

In the mask system of the embodiments of the present application, the fabric 20 is formed from yarns on at least a portion of the outer surface of the structural member, and the fabric 20 has the advantages of good comfort and breathability. In this way, the fabric 20 and the structural member as a whole are structurally stable and provide corresponding functions, and can also provide a user with good comfort and breathability when the structural member is in contact with the human body via the fabric 20, thereby improving user satisfaction. There is no protruding interface for the fabric 20 in the mask system, thereby avoiding discomfort to the face or the head of the user. Moreover, when the structural member has a complex shape, the yarns can still be formed into a fabric on the outer surface of the structural member, so that the shape of the mask system and the shape of the structural member can be made very complex to meet the styling and use requirements of the mask system. The mask system also has the advantages of low production costs, reduced process difficulty, easier manufacturability, and suitability for mass production.

An embodiment of the present application provides a ventilation device, including a ventilator and a mask system, where the ventilator is connected to the mask system, and the mask system is a mask system described above.

Ventilators, as an effective means capable of artificially replacing a function of spontaneous ventilation, can be used in respiratory failure, anaesthesia and respiration management during major surgery, respiratory support therapy and emergency resuscitation caused by various causes. The ventilators are vital medical devices that can function to prevent and treat respiratory failure, reduce complications, and save and extend patients' lives.

The ventilation device of the embodiments of the present application includes a ventilator, and a mask system connected to the ventilator. The mask system is configured to be worn on a human face and positioned at a relatively comfortable area of the face by means of a headstrap kit, thereby ensuring the user's comfort. Moreover, the ventilation device can implement conditioning, improvement and treatment functions.

Since the mask system has the yarns formed into a fabric on at least a portion of the outer surface of the structural member, the ventilation device of the embodiments of the present application has the advantages of low production costs, reduced process difficulty, easier manufacturability, and suitability for mass production. As a result, the ventilation device has the advantages of low production costs and suitability for mass production.

An embodiment of the present application provides a mask manufacturing method, which is applied to the mask system described above. As shown in Fig. 8, the manufacturing method includes the following steps.

In step S01, a structural member is provided.

In this step, the structural member has certain functions, which are not specifically limited in the embodiments of the present application. The structural member is a component, a ventilation headstrap tube 10, a strut arm, etc. For another example, the structural member is used for ventilation, support, connection to a gas delivery tube, etc.

In step S02, yarns are formed into a fabric on at least a portion of an outer surface of the structural member.

In this step, the yarns are formed into a fabric on the entire outer surface of the structural member, or the yarns are formed into a fabric on a portion of the outer surface of the structural member, depending on specific usage requirements. For example, when the structural member is a ventilation headstrap tube 10, referring to Fig. 3, it shows a structural schematic view of the yarns formed into a fabric on the entire outer surface of the ventilation headstrap tube 10; and referring to Fig. 7, it shows a structural schematic view of the yarns formed into a fabric on a portion of the outer surface of the ventilation headstrap tube 10.

In this step, a plurality of yarns are formed into the fabric 20 after establishing a stable relationship. The method for forming yarns into a fabric can be selected according to usage requirements. For example, the yarns are knitted on the structural member, and the fabric 20 is a knitted member. For another example, the yarns are woven on the structural member, and the fabric 20 is a woven member.

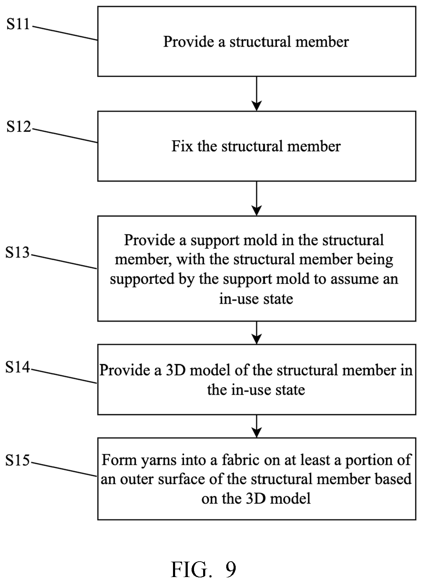

Optionally, an embodiment of the present application provides another mask manufacturing method. As shown in Fig. 9, the manufacturing method includes the following steps.

In step S11, a structural member is provided.

This step is the same as step S01 described above, which will not be repeated in this embodiment of the present application.

In step S12, the structural member is fixed.

In this step, the structural member is fixed to ensure that the position of the structural member is not moved during subsequent forming of yarns into a fabric.

Optionally, in an embodiment, the yarns are formed into a fabric by means of a fabric forming device. In this case, the structural member is fixed to a worktable of the fabric forming device.

In step S13, a support mold is provided in the structural member, with the structural member being supported by the support mold to assume an in-use state.

In this step, when the structural member is a member whose shape differs between a non-use state and an in-use state, such as the ventilation headstrap tube 10, the structural member needs to be supported, such that the structural member assumes the shape of the in-use state, thereby allowing the fabric to conform to the shape of the structural member in the in-use state.

The function of the support mold is to provide support such that the structural member assumes the in-use state.

In this step, when the material properties of the structural member are such that its shape remains the same in both the non-use state and the in-use state, this step can be omitted.

In step S14, a 3D model of the structural member in the in-use state is provided.

In this step, the 3D model of the structural member in the in-use state is provided, and the 3D model is used for subsequent forming of yarns into a fabric. The 3D model is configured to be input into a controller of the fabric forming device.

In step S15, yarns are formed into a fabric on at least a portion of an outer surface of the structural member based on the 3D model.

In this step, the fabric forming device forms the fabric based on the 3D model such that the yarns are formed into the fabric on at least a portion of the outer surface of the structural member. 3D software may be used for modelling. The 3D modelling data is input into the controller of a knitting device or a weaving device, and the knitting device or the weaving device operates based on the 3D modelling data to complete the knitting or weaving of the yarns.

In the controller, different thicknesses, densities and fabric forming methods may be set in different sections, so that different areas have different breathability. According to the actual requirements of the mask system, different knitting or weaving stiffnesses may be set, to achieve the purpose of increasing the rigidity of a part by means of the fabric.

In this step, the tension of the knitting or weaving may be set according to the material properties of the structural member to be knitted or woven, so as to ensure that the structural member is not affected by the fabric during normal operation.

It should be noted that in this specification, relational terms such as "first" and "second" are only used to distinguish one entity or operation from another entity or operation, and do not necessarily require or imply that there is any such actual relationship or order between these entities or operations. Moreover, the terms "comprise", "include", or their any other variants are intended to cover a non-exclusive inclusion, so that a process, a method, an article, or a device that includes a list of elements not only includes those elements but also includes other elements which are not expressly listed, or further includes elements inherent to such process, method, article, or device. If no more limitations are made, an element limited by "include a/an..." does not exclude other same elements existing in the process, the method, the article, or the device which includes the element.

The embodiments in the specification are all described in a relevant manner, mutual reference may be made to the same or similar parts of the embodiments, and each embodiment focuses on description of differences from other embodiments. As the embodiments of an apparatus, an electronic device, a computer-readable storage medium and a computer program product containing instructions are substantially similar to method embodiments, their descriptions are relatively concise, and for relevant details, reference may be made to the corresponding descriptions in the method embodiments.

The foregoing descriptions are merely illustrative of the preferred embodiments of the present invention but not intended to limit the scope of the present invention. Any modifications, equivalent substitutions, improvements, etc. made within the spirit and principles of the present invention shall fall into the scope of protection of the present invention.

## Claims

1. A mask system, **characterized by** comprising a fabric and at least one structural member, wherein
the structural member is at least a partial structure of the mask system, and at least a portion of an outer surface of the structural member is disposed opposite to a human skin in an in-use state; and
the fabric is formed from yarns on at least a portion of the outer surface of the structural member, and the structural member is in contact with the human skin via the fabric.

2. The mask system according to claim 1, **characterized in that** the structural member comprises a first support portion configured to be in contact with a human face; and the fabric wraps around an outer surface of the first support portion.

3. The mask system according to claim 1 or 2, **characterized in that** the structural member comprises a connecting portion configured to be connected to an external member; and the fabric comprises a clearance opening for providing clearance for the connecting portion.

4. The mask system according to claim 3, **characterized in that** the connecting portion is at least one of a ventilation port, an ear strap portion, a connection port and an electrical interface.

5. The mask system according to any one of claims 1 to 4, **characterized in that** the structural member is at least one of a silicone member and a plastic member.

6. The mask system according to any one of claims 1 to 5, **characterized in that** the structural member is at least one of a bracket, a frame and a ventilation headstrap tube.

7. The mask system according to any one of claims 1 to 6, **characterized in that** the fabric is a knitted member.

8. The mask system according to any one of claims 1 to 6, **characterized in that** the fabric is a woven member.

9. A ventilation device, **characterized by** comprising a ventilator and a mask system, wherein the ventilator is connected to the mask system, and the mask system is a mask system of any one of claims 1 to 8.

10. A mask manufacturing method for use in manufacturing a mask system of any one of claims 1 to 8, **characterized by** comprising:
providing a structural member; and
forming yarns into a fabric on at least a portion of an outer surface of the structural member.

11. The mask manufacturing method according to claim 10, **characterized in that** providing a structural member comprises:
fixing the structural member; and
providing a support mold in the structural member, with the structural member being supported by the support mold to assume an in-use state.

12. The mask manufacturing method according to claim 11, **characterized in that** fixing the structural member comprises:
fixing the structural member to a worktable of a fabric forming device, the fabric forming device being configured to form the yarns into a fabric.

13. The mask manufacturing method according to claim 10, **characterized in that** forming yarns into a fabric on at least a portion of an outer surface of the structural member comprises:
providing a 3D model of the structural member in an in-use state; and
forming yarns into a fabric on at least a portion of an outer surface of the structural member based on the 3D model.

14. The mask manufacturing method according to claim 10, **characterized in that** the yarns are knitted on at least a portion of the outer surface of the structural member.

15. The mask manufacturing method according to claim 10, **characterized in that** the yarns are woven on at least a portion of the outer surface of the structural member.
